# EUROPEAN PATENT SPECIFICATION

(11) **EP 2 437 048 B1**
(45) Date of publication and mention of the grant of the patent: **06.01.2016**
(21) Application number: 10780078.1
(22) Date of filing: 05.07.2010
(51) Int. Cl.: G01N 33/543, G01N 33/533, G01N 33/58, G01N 33/553

(54) **APPLICATION OF GOLD NANOPARTICLES BONDED DIRECTLY TO LUMINOL IN IMMUNOASSAY**
ANWENDUNG VON DIREKT AN LUMINOL GEBUNDENEN GOLDNANOPARTIKELN IN EINEM IMMUNOASSAY
APPLICATION DE NANOPARTICULES D'OR LIÉES DIRECTEMENT À DU LUMINOL DANS UN IMMUNOESSAI

(30) Priority: 27.05.2009 CN 200910085655
(43) Date of publication of application: 04.04.2012
(73) Proprietor: University of Science and Technology of China, Anhui 230026 (CN)
(72) Inventor: CUI, Hua, Anhui 230026 (CN); TIAN, Dayong, Anhui 230026 (CN)
(74) Representative: Yeadon IP Limited
(86) International application number: PCT/CN2010/074984
(87) International publication number: WO 2010/135997

(56) References cited:
- CN-A- 101 113 980
- CN-A- 101 148 584
- US-A1- 2004 058 389
- US-A1- 2007 275 383
- US-A1- 2007 275 383
- CUI, H.; WANG, W.; DUAN, C. F.; DONG, Y. P.; GUO, J. Z.: "Synthesis, Characterization, and Electrochemiluminescence of Luminol Reduced Gold Nanoparticles and Their Application in a hydrogen Peroxide Sensor", CHEM. EUR. J., vol. 13, 2007, page 6975, XP8148624,
- TIAN D ET AL: "Sandwich-type electrochemiluminescence immunosensor based on N-(aminobutyl)-N-ethylisoluminol labeling and gold nanoparticle amplification", TALANTA, ELSEVIER, AMSTERDAM, NL, vol. 78, no. 2, 30 April 2009 (2009-04-30) , pages 399-404, XP025914317, ISSN: 0039-9140, DOI: 10.1016/J.TALANTA.2008.11.037 [retrieved on 2008-12-03]
- CUI, HUA ET AL.: 'Synthesis, characterization, and electrochemiluminescence of luminol-reduced gold nanoparticles and their application in a hydrogen peroxide sensor' CHEM. EUR. J. vol. 13, 2007, pages 6975 - 6984, XP008148624
- TIAN, DAYONG ET AL.: 'Sandwich-type electrochemiluminescence immunosensor based on N-(aminobutyl)-N-ethylisoluminol labeling and gold nanoparticle amplification' TALANTA vol. 78, 03 December 2008, pages 399 - 404, XP025914317
- DAI, LU ET AL.: 'Detecting estrogens based on the catalysis of Au-Ag composite nanoparticles to lumenol-H202 CL system' JOURNAL OF ANHUI NORMAL UNIVERSITY vol. 32, no. 1, January 2009, pages 45 - 50, XP008148603
- HUANG, LIZHANG ET AL.: 'Study on the electrochemiluminescence oflumenol using nano-Au/multi-wall carbon nanotube modified electrode' JOURANL OF ANALYTICAL SCIENCE vol. 25, no. 1, February 2009, pages 47 - 50, XP008148602
- STEPHANE ROUX ET AL.: 'Synthesis, characterization of dihydrolipoic acid capped gold nanoparticles, and functionalization by the electroluminescent luminol' LANGMUIR vol. 21, 2005, pages 2526 - 2536, XP002526780
- TIAN, DAYONG ET AL.: 'Ultrasensitive electrochemiluminescence immunosensor based on luminol functionalized gold nanoparticel labeling' BIOSENSORS AND BIOELECTRONICS vol. 25, 17 March 2010, pages 2290 - 2295, XP008148648
- YANG, XIAOYAN ET AL.: 'Luninol/ antibody labeled gold nanoparticles for chemiluminescence immunoassay of carcinoembryonic antigen' ANALYTICA CHIMICA ACTA vol. 666, 04 April 2010, pages 91 - 96, XP008148649

## Description

The present application claims the priority of China Patent Application No. 200910085655.2 filed on May 27, 2009, entitled "Application of Luminol Directly Bonded Gold Nanoparticles in Immunoassay".

### Field of the Invention

The present invention relates to the use of luminol directly bonded gold nanoparticles, particularly to the use thereof in immunoassays.

### Background of the Invention

Chemiluminescence is the light emission during a chemical reaction. As a means for light detection, chemiluminescence has been widely used in immunoassays due to its several advantages such as the independency on light sources, high sensitivity, wide range, low background noise, simple device, low cost. (Wu, A. H. B. Clin. Chim. Acta 2006, 369, 119; Zhan, W.; Bard, A. J. Anal. Chem. 2007, 79, 459 ; Yin, X. B.; Qi, B.; Sun; X. P.; Yang; X. R.; Wang; E. K. Anal. Chem. 2004, 77, 3525; Jie, G. F.; Huang, H. P.; Sun, X. 1.; Zhu, J. J. Biosens. Bioelectron. 2008, 23,1896; Egashira, N.; Morita, S.; Hifumi, E.; Mitoma, Y.; Uda, T. Anal. Chem. 2008, 80, 4020). At present, chemiluminescence immunoassay has become a major tool of clinical analysis in most large hospitals. The performance of a chemiluminescence immunoassay often depends on the probe used. The existing commercially available probes for chemiluminescence immunoassay can be divided into two types: (a) chemiluminescent molecules, such as isoluminol and derivatives thereof, acridine esters, ruthenium bipyridine, etc. The chemiluminescent molecule probe based immunoassays often involve not only the condensation reaction to bind the chemiluminescent molecules to biological molecules, but also the further purification of the reaction product, which is complicated, tedious and time-consuming. (b) Enzymes, such as peroxidases, alkaline phosphatases, etc. A series of chemiluminescence enzyme immunoassays for biological analysis have been established by utilizing the catalytic effect of enzyme molecules on particular chemiluminescent reactions. These methods bear the disadvantage that the enzyme molecules are easily inactivated, resulting in the poor stability of the analysis. Moreover, the above-mentioned analytical techniques using luminescent molecules and enzymes based probes suffer from additional disadvantages, such as high cost of analysis, difficulties in detecting analytes at ultra-trace amount and difficulties in performing a rapid, *in situ* analysis.

Recently, nanomaterials have been utilized as probes in chemiluminescent immunoassay due to their unique biocompatibility, surface properties, and chemiluminescent properties. Gold nanoparticles have been used as probes in chemiluminescent immunoassays because of their excellent stability and biocompatibility. In 2005, Lu's group and Li's group both developed gold nanoparticle labeled, "stripping" chemiluminescent immunoassays based on the dissolving/oxidation of gold nanoparticles to trivalent gold ion Au (III) after immunoreactions, which could react with luminol to produce chemiluminescence signals through the catalytic or oxidative effect of Au (III) on luminol chemiluminescent system (Fan, A. P.; Lau, C. W.; Lu, J. Z. Anal. Chem. 2005, 77, 3238; Li, Z. F.; Wang, Y. C.; Liu, C. H.; Li, Y K. Anal. Chim. Acta 2005, 551, 85). The main disadvantage of the method is the extremely caustic conditions for the stripping of gold nanoparticles (such as high concentration of HNO₃-HCl, or toxic HBr-Br₂), resulting in the high background of chemiluminescence. Thereafter, two groups successively reported immunoassay methods in which gold nanoparticles are directly used as catalytic probes (Wang, Z. P.; Hu, J. Q.; Jin, Y.; Yao, X.; Li, J. H. Clin. Chem. 2006, 52, 1958 ; Duan, C. F.; Yu, Y Q.; Cui, H. Analyst 2008, 133, 1250). The luminescent detection is directly carried out through catalytic effect of gold nanoparticles on the chemiluminescence reaction without the "stripping" process. Li's group used irregular gold nanoparticles as labels to develop a chemiluminescence immunoassay, in which irregular gold nanoparticles as catalytic labels directly enhances the chemiluminescence of luminol-peroxide hydrogen system. Although the caustic stripping procedure is avoided in this protocol, the synthesis of such irregular gold nanoparticles is hard to control, requiring long period reaction with temperature control (40°C, 24 h), purging of oxygen and relatively low monodispersity, which may influence the repeatability among different batches, limiting the practical application of this method. Duan et al. reported that regular spherical gold nanoparticles with a diameter of 8-68 nm could induce rapid and strong light emission at 425 nm in luminol-silver nitrate system (Duan, C. F.; Yu, Y. Q.; Cui, H. Analyst 2008, 133, 1250). Based on this discovery, a microplate-compatible, non-stripping chemiluminescence immunoassay protocol with citrate-protected, conventional gold nanoparticles as labels based on gold nanparticles-catalyzed luminol-AgNO₃ chemeluminescence system was developed for determining human IgG. Compared with the reported chemiluminescent immunoassays based on gold nanoparticles, this method avoids the caustic stripping process and the synthesis of irregular gold nanoparticles, improving its applicability in clinical diagnosis. However, this gold-nanoparticle-based chemiluminescent immunoassay has a detection limit of 0.5-100 ng/mL for IgG, which need to be further improved. In addition, the luminescent reagents have to be separately introduced after the immunoreaction, resulting in a limitation in the application thereof. Therefore, it is highly desired to develop novel immunoassay probes and new techniques for chemiluminescent immunoassays to overcome these disadvantages.

Luminol, also called 3-aminophthalhydrazide, with the chemical formula of C₈H₇N₃O₂, appears as yellow crystals or beige powders at room temperature, and is a relatively artificially synthesized stable organic compound. Being simple in structure, easy to synthesize, and soluble in water, luminol is one of the most widely used chemiluminescent agents. Luminol *per se* is not suitable to be directly used as probes for immunoassay as when luminol is labeled with antigen or antibody molecules by its aromatic amine group, the steric hindrance would increase and luminescent efficiency would decrease. However, luminol is often used as luminescent substrates in enzyme-labeled chemiluminescence immunoassays.

Recently, it has been reported that luminol can be bonded to the surface of gold nanoparticles through chemical reactions to form luminol-bonded gold nanoparticles (Cui, H.; Wang, W.; Duan, C. F.; Dong, Y. P.; Guo, J. Z. Chem. Eur. J. 2007, 13, 6975; Roux, S.; Garcia, B.; Bridot, J. L.; Salom, Marquette, C. Langmuir. 2005, 21, 2526), and the luminol-bonded gold nanoparticles are of electrochemiluminescence activity. However, the application thereof in biological analysis has not been studied. In 2005, Roux et al. (Roux, S.; Garcia, B.; Bridot, J. L.; Salom, Marquette, C. Langmuir. 2005, 21, 2526) used dihydro-lipoic acid as protection reagent in the reduction of chloroauric acid (HAuCl₄.3H₂O) by NaBH₄ to synthesize gold nanoparticles protected by dihydro-lipoic acid. The carboxyl groups of dihydro-lipoic acid on the surface of gold nanoparticles were activated by 1-ethyl-3-(3-dimethylaminopropyl)-carbodiimide (EDC) and N-hydroxylsuccinimide (NHS), and then luminol is grafted on the surface of gold nanoparticles through the condensation reaction between the -NH₂ of luminol and the -COOH of dihydro-lipoic acid. This process requires several steps to obtain the luminol bonded gold nanoparticles including multiple-step reactions, washing of the precipitate, vacuum distillation, filtration, dispersion, etc. Thus it is laborious and time-consuming. It is demonstrated that luminol bonded to the surface of gold nanoparticles through dihydro-lipoic acid maintains considerable electrochemiluminescence activity. However, it has not been studied whether or not such luminol bonded gold nanoparticles can be linked with biological molecules such as proteins and DNAs, or whether or not the obtained complex bound with biological molecules still maintain activity in chemiluminescence and electrochemiluminescence. For luminol-bonded gold nanoparticles obtained by this method, the surface thereof is largely covered by dihydro-lipoic acid and luminol molecules. It is assumed that it is difficult to directly link proteins or DNAs to the surface of gold nanoparticles or luminol molecules. On the other hand, the amount of dihydro-lipoic acid linked proteins and DNAs will be very limited, since most of active sites of dihydro-lipoic acid have been occupied by luminol and gold nanoparticles. In addition, it is difficult to maintain a high luminescent activity of luminol upon linkage. In 2007, Our group found that chloroauric acid could be directly reduced by luminol through one-step synthesis to form luminol directly bonded gold nanoparticles (Cui, H.; Wang, W.; Duan, C. F.; Dong, Y. P.; Guo, J. Z. Chem. Eur. J. 2007, 13, 6975). Further characterization demonstrated that luminol molecules are coated on the surface of gold nanoparticles through the weak covalent interaction between gold and nitrogen atoms. The gold nanoparticles directly bonded with luminol were then assembled on the surface of Au electrode by virtue of the electrostatic interaction between bridging molecules cysteine and gold nanoparticles. It was found that the modified electrode was of electrochemiluminescence activity in alkaline solutions, and the intensity increased with the concentration of H₂O₂. As a result, an electrochemiluminescence sensor for H₂O₂ was developed. However, the sensitivity for the determination of H₂O₂ is poor, with the detection limit of only 1×10⁻⁷ mol/L of H₂O₂. In addition, the stability of this analytical method is not satisfying and it is difficult to use it in practical determination. In above mentioned work, the applications of luminol directly bonded gold nanoparticles in biological analysis, such as immunoassay, have not been studied.

### Summary of the Invention

The present invention discovers that luminol directly bonded gold nanoparticles, which is obtained through one-step reduction of chlorauric acid with luminol, can be efficiently linked to protein molecules, and the resulted complex has excellent luminescence activity and stability. Based thereon, the present invention provides the following three new uses of luminol directly bonded gold nanoparticles.

In the first aspect, there is provided a luminol directly bonded gold nanoparticle immunoprobe, wherein the immunoprobe is an antibody (polyclonal or monoclonal) or antigen or protein which is labeled with luminol directly bonded gold nanoparticles.

The luminol directly bonded gold nanoparticles may be obtained by the reduction of chlorauric acid with luminol in a one-step process.

Furthermore, the immunoprobe may be a chemiluminescence immunoprobe, in particular an electrochemiluminescence immunoprobe.

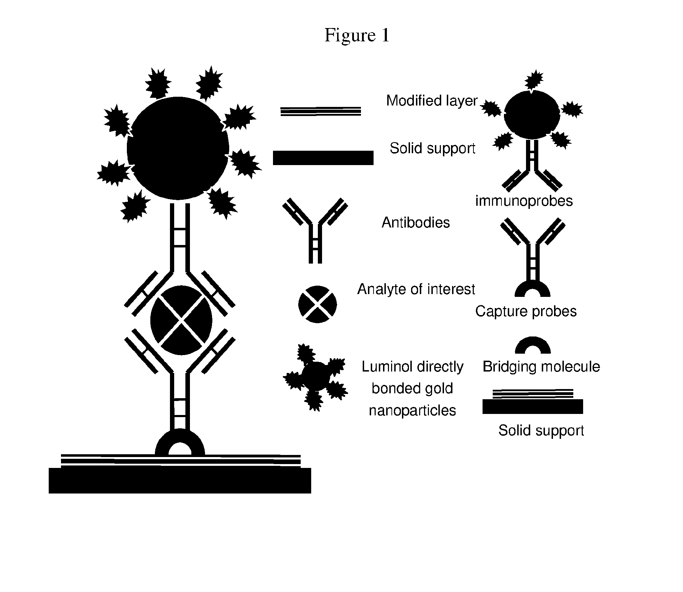

In a second aspect, there is provided a chemiluminescence immunoassay based on any one of the above-defined luminol directly bonded gold nanoparticle immunoprobes, the method comprising the following steps:
A, synthesizing the above-defined luminol directly bonded gold nanoparticle immunoprobe;
B, incubating a capture probe with a solid support, such that the capture probe is linked onto the solid support, forming a capture probe/solid support complex;
C, incubating the capture probe/solid support complex obtained in Step B with an analyte of interest, allowing the binding between the capture probe/solid support complex and the analyte of interest, forming an analyte of interest/capture probe/solid support complex;
D, incubating the analyte of interest/capture probe/solid support complex obtained in Step C with the luminol directly bonded gold nanoparticle immunoprobe obtained in Step A, so as to form an immunoassay probe/analyte of interest/capture probe/solid support sandwich complex as shown in Figure 1;
E, adding the immunoassay probe/analyte of interest/capture probe/solid support sandwich complex obtained in Step D into a chemiluminescence cuvette, adding a substrate solution to produce chemiluminescence, and then detecting the chemiluminescence using a chemiluminescence analyzer.

In the above-defined chemiluminescence immunoassay, the capture probe may be an antibody or an antibody linked to a bridging molecule. The bridging molecule may be biotin.

In the above-defined chemiluminescence immunoassay, the solid support may be selected from the group consisting of a magnetic bead, a microplate, nylon, glass and an electrode.

The above-defined magnetic bead may be a conventional magnetic bead or a magnetic bead modified with gold nanoparticles, wherein the conventional magnetic bead may be a magnetic bead of iron oxide coated by polystyrene. The microplate may be a conventional microplate or a microplate modified with gold nanoparticles, wherein the conventional microplate may be a polystyrene microplate. The nylon may be conventional nylon or nylon modified with gold nanoparticles, wherein the conventional nylon may be polyamide nylon. The glass may be conventional glass or glass modified with gold nanoparticles, wherein the conventional glass may be aldehydized, aminized, or thiolized glass. The glass modified with gold nanoparticles may be glass plated with gold or silver.

The chemiluminescence immunoassay may be an electrochemiluminescence immunoassay. Here the solid support may be an electrode.

The electrode may be an electrode modified with gold nanoparticles. The electrode modified with gold nanoparticles may be a gold electrode modified with gold nanoparticles, a platinum electrode modified with gold nanoparticles, a vitreous carbon electrode modified with gold nanoparticles, a graphite electrode modified with gold nanoparticles, a wax-impregnated graphite electrode modified with gold nanoparticles, or an indium-tin oxide electrode modified with gold nanoparticles.

The bridging molecules used with the gold electrode modified with gold nanoparticles may be a double-thiol compound or a compound carrying an amino group and a thiol group. The double-thiol compound may be 1,3'-propanedithiol.

The gold nanoparticles on the solid support may be linked to streptavidin. The particle size of gold nanoparticles may be 10-38 nm, preferably 16 nm.

In the cases that the chemiluminescence immunoassay is an electrochemiluminescence immunoassay, the chemiluminescence cuvette in Step E may be an electrochemiluminescence cuvette, in which the sandwich immune complex may produce chemiluminescence upon addition of a substrate solution and application of potential.

The substrate solution may be carbonate buffer containing peroxide hydrogen.

The applied working potential may be pulse potential, cyclic voltammetry, or linear voltammetry. The pulse potential may be two-phase pulse potential.

According to the prior art, the solid support in the electrochemiluminescence immunoassay may also be an electrode linked to magnetic beads.

In the chemiluminescence immunoassay, the analyte of interest may be an antibody, an antigen, or a protein. The sample containing the analyte of interest may be selected from the group consisting of a clinical sample, a drug sample, a water sample, a biological sample, an air sample, a food sample and a beverage sample.

In the third aspect, there is provided a kit for performing the above-defined chemiluminescence immunoassay, the kit comprising:
a. a luminol directly bonded gold nanoparticle immunoprobe, as defined above;
b. a capture probe;
c. a solid support;
d. a substrate solution;
e. a washing solution.

The luminol directly bonded gold nanoparticle immunoprobe may be an antibody labeled with luminol directly bonded gold nanoparticles. The particle size of gold nanoparticles in the probes may be 14-25 nm, preferably 25 nm.

The capture probe may be an antibody labeled with biotin.

The solid support may be a gold electrode modified with gold nanoparticles. The gold electrode modified with gold nanoparticles may be prepared by assembling gold nanoparticles on a gold electrode via 1,3'-propanedithiol. The gold nanoparticles may be gold nanoparticles linked to streptavidin. The particle size of gold nanoparticles may be 10-38 nm, preferably 16 nm.

The substrate solution may comprise any buffer containing any reagent which may react with luminol and any substance which is capable of enhancing the chemiluminescence. In particular, the substrate solution may be carbonate buffer containing peroxide hydrogen, in which the carbonate may consist of sodium carbonate and sodium bicarbonate. The concentration of peroxide hydrogen may be 1 mmol/L. The concentrations of sodium carbonate and sodium bicarbonate may each be 0.02 mol/L.

The washing solution may be phosphate buffer, comprising potassium dihydrogen phosphate, disodium hydrogen phosphate, sodium chloride and potassium chloride. The concentration of phosphate buffer may be 0.02 mol/L, in which the concentration of potassium dihydrogen phosphate may be 0.2 g/L, the concentration of disodium hydrogen phosphate may be 2.9 g/L, the concentration of sodium chloride may be 0.8 g/L and the concentration of potassium chloride may be 0.2 g/L.

The luminol directly bonded gold nanoparticle immunoprobe, the assays, and kit according to the present invention are useful in the determination of various analytes in various samples, in the art of clinical analysis, pharmaceutical analysis, food safety assessment, environmental monitoring, and the like. Examples of the analyte include, but not limit to, microbes (such as viruses, bacteria, fungi), proteins, antibodies, antigens, biological toxins, chemical toxins, and the like. Examples of the sample include, but not limit to, clinical samples, drug samples, water samples, biological samples, air samples, food samples, and beverage samples, etc.

The following advantages are achieved by the present invention, as compared to the prior art.
(1) It is the first time that the luminol directly bonded gold nanoparticles are used as probes for immunoassays, suggesting a new-generation labeling technique for immunoassay.
(2) The luminol directly bonded gold nanoparticles are obtained by a one-step method through reduction of chlorauric acid with luminol. The method for preparing the analytical probe, in which the gold nanoparticle is used as luminescent label for antibody, is simple and fast, easy to perform with low-cost. The proposed method overcomes the disadvantages of the existing luminescent reagent based labeling techniques, which are complicated, time-consuming and expensive.
(3) Due to the good biocompatibility of gold nanoparticles, labeling antibodies with gold nanoparticles maintains a maximum activity of the antibodies.
(4) Luminol *per se* is not suitable to be used as probes for immunoassay, since steric hindrance increases and luminescent efficiency reduces after antigen or antibody molecules are labeled with luminol by its aromatic amine. It is shown in the present invention that luminol bonded to the surface of gold nanoparticles is capable to directly label an antigen or an antibody, to produce an immunoprobe with high luminescent efficiency and stability. The luminol directly bonded gold nanoparticle immunoprobe according to the present invention are of great commercial value, since luminol is much cheaper than common chemiluminescent labeling reagent such as 4-aminobutylethyl isoluminol (ABEI), acridinium esters and ruthenium bipyridine, etc., and the present labeling method is also simple and easy to carry out.
(5) The chemiluminescence immunoassay based on luminol directly bonded gold nanoparticle immunoprobes provides amplification effect by dual layer of gold nanoparticles, lowering the detection limit, and thereby resulting in high sensitivity of the method. The experiments demonstrated that the detection limit for human IgG is as low as 1.0 pg/mL, which is about 1-5 orders of magnitude lower than the existing sandwich chemiluminescence immunoassays using gold nanoparticles and luminescent reagents as labels. Besides, the present method is easy, highly reproducible, and cost-effective.

### Description of the Drawings

Figure 1 is a scheme of the sandwich immune complex formed by the capture probe linked on a solid support, the analyte of interest and the analytical probe in the chemiluminescence immunoassay provided in the present invention;
Figure 2 is a scheme of the preparation process of the modified electrodes in the chemiluminescence immunoassay provided in the present invention;
Figure 3 shows the comparison of electrochemiluminescence signals from bare gold electrodes, 1,3'-propanedithiol modified gold electrodes, streptavidin/gold nanoparticles/1,3'-propanedithiol modified gold electrodes, and secondary antibody labeled with luminol directly bonded gold nanoparticles/antigen/primary antibody/biotin/streptavidin/gold nanoparticles/1,3'-propanedithiol modified gold electrodes.

The reference signs in the figure are as follows:
a. bare gold electrodes;
b. 1,3'-propanedithiol modified gold electrodes;
c. streptavidin/gold nanoparticles/1,3'-propanedithiol modified gold electrodes;
d. secondary antibody labeled with luminol directly bonded gold nanoparticles / antigen / primary antibody / biotin / streptavidin / gold nanoparticles / 1,3'-propanedithiol modified gold electrodes, as prepared in Example 2.

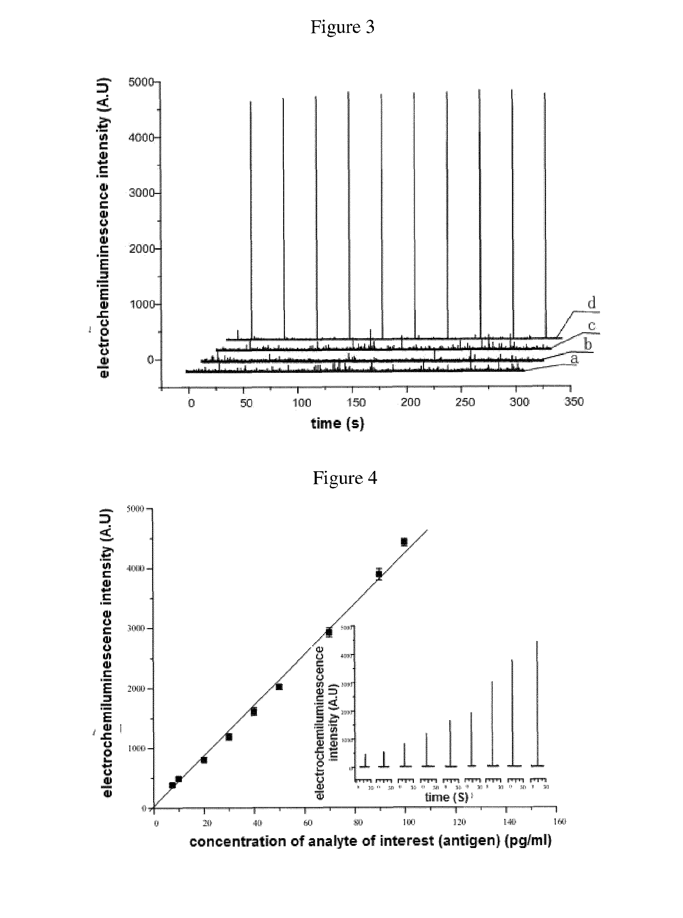

Figure 4 shows working curve for the determination of human IgG concentration by the chemiluminescence immunoassay in Example 3. The insert shows the electrochemiluminescence intensity corresponding to different human IgG concentrations.

### DETAILED DESCRIPTION OF EMBODIMENTS

The following section describes in detail the luminol directly bonded gold nanoparticle immunoprobes, the chemiluminescence immunoassay involving the luminol directly bonded gold nanoparticle immunoprobes, the corresponding kits and the use thereof.
1. A luminol directly bonded gold nanoparticle probe. The immunoprobe is an antibody or antigen labeled with luminol directly bonded gold nanoparticles, for example a polyclonal or monoclonal antibody or any other proteins labeled with luminol directly bonded gold nanoparticles. The luminol directly bonded gold nanoparticle is obtained in a one-step process by the reduction of chlorauric acid with luminol. The immunoprobe is a chemiluminescence immunoprobe, such as an electrochemiluminescence immunoprobe.
   The preparation of the luminol directly bonded gold nanoparticle immunoprobe according to the present invention comprises the following steps:
   (1) 100 mL of 0.01 % (w/w) HAuCl₄ solution is heated to boil, and 1.5 - 2.0 mL of a solution containing 0.01 mol/L luminol and 0.01 mol/L NaOH is added under reflux with stirring. The reaction is kept boiling and stirred under reflux for 30 min. Then the heat source is removed and the reaction is stirred for at least additional 15 min, resulting in a gold nanoparticle solution with a particle diameter of 14-25 nm;
   (2) The synthesized gold nanoparticles solution is dialyzed against ultrapure water at room temperature for 2 days and the ultrapure water is exchanged once every 8 hours, resulting in the luminol directly bonded gold nanoparticles;
   (3) 0.5 mL of 1.0 mg/mL antibody is added to the dialyzed gold colloid solution in Step (2), for which pH has been adjusted to 8.0 using 0.1 mol/L NaOH. The solution is incubated at room temperature for 30 min, and then 5 % (w/w) bovine serum albumin is added to the solution with stirring for 5 min, to the final concentration of 1%. Finally the solution is centrifuged at 17120 * g for 45 min so as to remove unreacted reagents and weakly bonded luminol at the surface of gold nanoparticles. The precipitate is then dissolved in 0.1 mol/L pH 7.4 phosphate buffer containing 1 % (w/w) bovine serum albumin to obtain luminol directly bonded gold nanoparticle immunoprobes.
2. The chemiluminescence immunoassay of the invention comprises the following steps:
   A, synthesizing the above-mentioned luminol directly bonded gold nanoparticle immunoprobe;
   B, incubating a capture probe with a solid support, such that the capture probe is linked onto the solid support, forming a capture probe/solid support complex;
   C, incubating the capture probe/solid support complex obtained in Step B with an analyte of interest, allowing the binding between the capture probe/solid support complex and the analyte of interest, forming an analyte of interest/capture probe/solid support complex;
   D, incubating the analyte of interest/capture probe/solid support complex obtained in Step C with the luminol directly bonded gold nanoparticle immunoprobe obtained in Step A, so as to form an immunoassay probe/analyte of interest/capture probe/solid support sandwich complex as shown in Figure 1;
   E, adding the immunoassay probe/analyte of interest/capture probe/solid support sandwich complex obtained in Step D into a chemiluminescence cuvette, adding a substrate solution to produce chemiluminescence, and then detecting the chemiluminescence using a chemiluminescence analyzer.

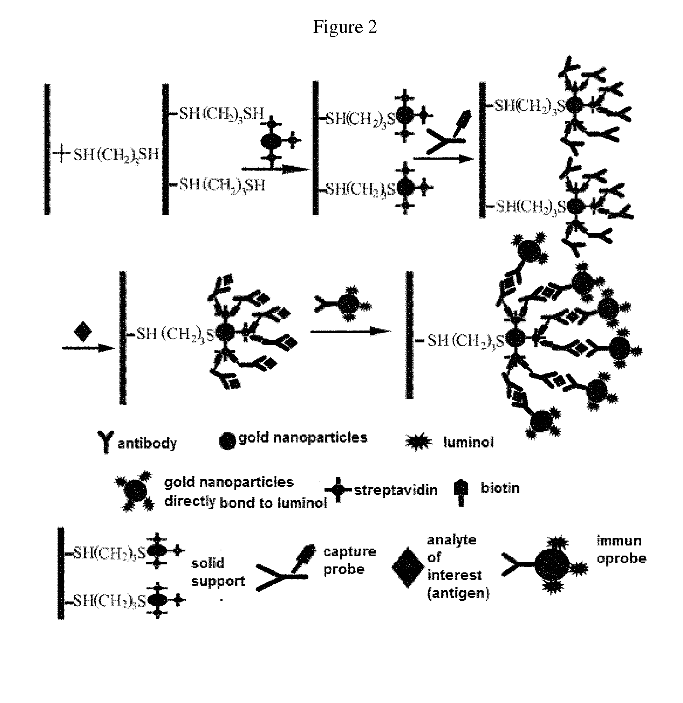

   The capture probe may be an antibody or an antibody linked to a bridging molecule. The antibody may be linked to the solid support by any known method. Examples of the solid support include, but not limit to, electrode materials, magnetic beads, microplates, nylon, glass and the like. The existing methods include, but not limit to, those reported in: Yin, X. B.; Qi, B.; Sun; X. P.; Yang; X. R.; Wang; E. K. Anal. Chem. 2004, 77, 3525 ; Wang, Z. P.; Hu, J. Q.; Jin, Y; Yao, X.; Li, J. H. Clin. Chem. 2006, 52, 1958; Jie, G. F.; Liu, B.; Pan, H. C.; Zhu, J. J.; Chen, G. N. Anal. Chem. 2007, 79, 5574; Zhan, W.; Bard, A. J. Anal. Chem. 2007, 79, 459; Yang, Z. J.; Xie, Z. Y.; Liu, H.; Yan, F.; Ju, H. X. Adv. Funct. Mater. 2008, 18, 3991; Miao, W. J.; Bard, A. J. Anal. Chem. 2003, 75, 5825.
   The solid support of the invention is preferably a solid support modified with gold nanoparticles. The capture probes are linked to the solid support by any known method for linking proteins with gold nanoparticles. The existing method include, but not limit to, those reported in: Yin, X. B.; Qi, B.; Sun; X. P.; Yang; X. R.; Wang; E. K. Anal. Chem. 2004, 77, 3525; Wang, Z. P.; Hu, J. Q.; Jin, Y; Yao, X.; Li, J. H. Clin. Chem. 2006, 52, 1958; Gonzalez-Garcia, M. B.; Fernandez-Sanchez, C.; Costa-Garcia, A. Biosens. Bioelectron. 2000, 15, 315. Streptavidin and biotin is preferable in the present invention. The streptavidin is linked to the gold nanoparticle, and biotin is linked to the antibody. The antibody is therefore grafted on the solid support modified with gold nanoparticles through the specific interaction between streptavidin and biotin.
   Furthermore, the chemiluminescence immunoassay is an electrochemiluminescence immunoassay. Here the solid support is an electrode. The electrode is preferably an electrode modified with gold nanoparticles, wherein the electrode modified with gold nanoparticles may be selected from the group consisting of gold electrodes modified with gold nanoparticles, platinum electrodes modified with gold nanoparticles, vitreous carbon electrodes modified with gold nanoparticles, graphite electrodes modified with gold nanoparticles, wax-impregnated graphite electrodes modified with gold nanoparticles, and indium-tin oxide electrodes modified with gold nanoparticles. Gold electrodes modified with gold nanoparticles are preferable in the present invention, wherein the bridging molecules used in the gold electrodes modified with gold nanoparticles include, but not limit to, double-thiol compounds and compounds carrying an amino group and a thiol group, etc.
   The gold electrode as described herein is modified with gold nanoparticles linked to streptavidin, which are commercially available and can be synthesized by established methods. The gold nanoparticle is protected by citrate salt, with a particle size within the range of 10-38 nm, and preferably 16 nm.
   The electrochemiluminescence immunoassay according to the present invention comprises the following steps:
   a, synthesizing any one of the above-mentioned luminol directly bonded gold nanoparticle immunoprobes;
   b, assembling gold nanoparticles linked to streptavidin onto a clean bare gold electrode, so as to obtain a streptavidin/gold nanoparticle modified gold electrode;
   c, incubating a capture probe containing biotin with the streptavidin/gold nanoparticle modified gold electrode obtained in Step B, so as to allow linkage between the capture probe and the gold nanoparticle modified electrode, obtaining a capture probe/biotin /streptavidin / gold nanoparticle modified gold electrode;
   d, incubating a sample containing the analyte of interest with the capture probe / biotin / streptavidin / gold nanoparticle modified gold electrode obtained in Step C, so as to link the analyte of interest to the capture probe, obtaining an analyte of interest / capture probe / biotin / streptavidin / gold nanoparticle modified gold electrode;
   e, incubating the immunoprobe obtained in Step a with the analyte of interest / capture probe / biotin / streptavidin / gold nanoparticle modified gold electrode obtained in Step d, so as to link the immunoprobe to the analyte of interest, obtaining an immunoprobe / analyte of interest / capture probe / biotin / streptavidin / gold nanoparticle modified gold electrode as shown in Figure 2;
   f, adding the immunoprobe / analyte of interest / capture probe / biotin / streptavidin / gold nanoparticle modified gold electrode obtained in Step e into a chemiluminescence cuvette, adding a substrate solution and applying potential, so as to obtain chemiluminescence, and then detecting the chemiluminescence using a chemiluminescence analyzer.

   The Step b is to modify the surface of a gold electrode by gold nanoparticles linked to streptavidin through double thiol compounds, wherein the double thiol compound is preferably 1,3'-propanedithiol.
   The substrate solution in Step f may be carbonate buffer containing peroxide hydrogen.
   The working potential applied in Step f may be pulse potential, cyclic voltammetry, or linear voltammetry. Pulse potential is preferable, and the pulse potential may be double-step pulse potential.
   The electrochemiluminescence immunoassay for determination of various analytes is illustrated as follows, in which a gold electrode modified with gold nanoparticles linked to streptavidin is used as the solid support, an antibody linked to biotin is used as the capture probe, and electrochemical methods are used to induce chemiluminesce.
   (1) Preparation of luminol directly bonded gold nanoparticle immunoprobes ;
      a-1. 100 mL of 0.01 % (w/w) HAuCl₄ solution is heated to boil, and 1.5 - 2.0 mL of a solution containing 0.01 mol/L luminol and 0.01 mol/L NaOH is added under reflux with stirring. The reaction is kept boiling and stirred under reflux for 30 min. Then the heat source is removed and the reaction, and the reaction stirred for at least additional 15 min, resulting in a gold nanoparticle solution with a particle diameter of 14-25 nm;
      b-1. The synthesized gold nanoparticles solution is dialyzed against ultrapure water at room temperature for 2 days and the ultrapure water is exchanged once every 8 hours, resulting in luminol directly bonded gold nanoparticles;
      c-1. 0.5 mL of 1.0 mg/mL antibody (secondary antibody) is added to the dialyzed gold colloid solution in Step b-1, for which pH has been adjusted to 8.0 using 0.1 mol/L NaOH. The solution is incubated at room temperature for 30 min, and then 5 % (w/w) bovine serum albumin is added to the solution with constant stirring for 5 min, to the final concentration of 1%. Finally the solution is centrifuged at 17120 * g for 45 min. The precipitate is then dissolved in 0.1 mol/L pH 7.4 phosphate buffer containing 1 % (w/w) bovine serum albumin obtaining luminol directly bonded gold nanoparticle immunoprobes.
   (2) Preparation of electrodes modified with gold nanoparticles
      a-2. A polished bare gold electrode is immersed into 2 mmol/L 1,3'-propanedithiol solution, and incubated at room temperature for 20 hours, then taken out and rinsed with ethanol and ultrapure water, resulting in 1,3'-propanedithiol modified gold electrode;
      b-2. Streptavidin coated gold nanoparticles are added dropwise on the 1,3'-propanedithiol modified gold electrode prepared in Step a-2, and the electrode is kept in dark at 4□ for 4 hours, then taken out and rinsed with ultrapure water, resulting in a streptavidin / gold nanoparticle / 1,3'-propanedithiol modified gold electrode;
   (3) Immobilization of sandwich complexes on the gold nanoparticle modified electrodes
      a-3. Biotinylated primary antibody is added dropwise on the streptavidin / gold nanoparticle / 1,3'-propanedithiol modified gold electrode obtained in Step (2), the electrode is kept at 37 °C for 60 min, and rinsed with 0.02 mol/L pH=7.4 phosphate buffer, resulting in a primary antibody / biotin / streptavidin / gold nanoparticle / 1,3'-propanedithiol modified gold electrode. Subsequently, 1% (w/w) bovine serum albumin is added dropwise on the primary antibody / biotin / streptavidin / gold nanoparticle / 1,3'-propanedithiol modified gold electrode, and the electrode is kept at 37 °C for 40 min, then rinsed with 0.02 mol/L pH=7.4 phosphate buffer.
      b-3. An antigen as the analyte of interest is added dropwise on the primary antibody / biotin / streptavidin / gold nanoparticle / 1,3'-propanedithiol modified gold electrode obtained in Step a-3, and the electrode is kept at 37 °C for 60 min, then rinsed with 0.02 mol/L pH=7.4 phosphate buffer, resulting in an antigen / primary antibody / biotin / streptavidin / gold nanoparticle / 1,3'-propanedithiol modified gold electrode.
      c-3. The luminol directly bonded gold nanoparticles labeled secondary antibody obtained in Step (1) (i.e. the immunoprobe) is added dropwise to the antigen / primary antibody / biotin / streptavidin / gold nanoparticle / 1,3'-propanedithiol modified gold electrodes obtained in Step b-3, kept at 37 °C for 60 min and the electrode is rinsed with 0.02 mol/L pH=7.4 phosphate buffer, resulting in a modified electrode on which the luminol directly bonded gold nanoparticles labeled secondary antibody specifically binds to analyte antigen, i.e. the luminol directly bonded gold nanoparticles labeled secondary antibody / antigen / primary antibody / biotin / streptavidin / gold nanoparticle / 1,3'-propanedithiol modified gold electrode as shown in Figure 2.
   (4) Electrochemiluminescence immunoassay The luminol directly bonded gold nanoparticles labeled secondary antibody / antigen / primary antibody / biotin / streptavidin / gold nanoparticle / 1,3'-propanedithiol modified gold electrode prepared in (3) is put into an electrochemiluminescence cell, and 0.02 mol/L pH=9.95 carbonate substrate solution containing 1 mmol/L peroxide hydrogen is added. Double-step pulse potential is applied with the pulse potential of 0.8 V, the pulse period of 30 s, the pulse time of 0.1 s, and the initial potential of 0 V, chemiluminescence is produced and the signal is detected by a luminescence analyzer.
      The primary antibody labeled with biotin in Step (3)a-3 may be particularly goat-anti-human IgG labeled with biotin.
      In Step (3)b-3, the antigen may be particularly human IgG.
      In Step (3)c-3, the secondary antibody labeled with luminol directly bonded gold nanoparticles may be particularly goat-anti-human IgG labeled with luminol directly bonded gold nanoparticles.
3. The present invention also relates to a kit for the chemiluminescent immunoassay, the kit comprising:
   A'. any luminol directly bonded gold nanoparticle immunoprobe as described above;
   B'. a capture probe;
   C'. a solid support;
   D'. a substrate solution;
   E'. a washing solution.

The luminol directly bonded gold nanoparticle immunoprobe is an antibody labeled with luminol directly bonded gold nanoparticles. The particle size of the gold nanoparticles in the immunoprobe may be within the range of 14-25 nm, preferably 25 nm.

The capture probe may be an antibody labeled with biotin.

The solid support may be a gold electrode modified with gold nanoparticles. The gold electrode modified with gold nanoparticles is prepared by assembling gold nanoparticles onto a gold electrode via 1,3'-propanedithiol. The gold nanoparticles may be gold nanoparticles linked to streptavidin. The particle size of gold nanoparticles may be within the range of 10-38 nm, preferably 16 nm.

The substrate solution comprises any buffer containing any reagent which may react with luminol and any substance which is capable of enhancing the chemiluminescence. In particular, the substrate solution may be carbonate buffer containing peroxide hydrogen, in which the carbonate consists of sodium carbonate and sodium bicarbonate. The concentration of peroxide hydrogen is 1 mmol/L. The concentrations of sodium carbonate and sodium bicarbonate are each 0.02 mol/L.

The washing solution is phosphate buffer, comprising potassium dihydrogen phosphate, disodium hydrogen phosphate, sodium chloride and potassium chloride. The concentration of phosphate buffer is 0.02 mol/L, in which the concentration of potassium dihydrogen phosphate is 0.2 g/L, the concentration of disodium hydrogen phosphate is 2.9 g/L, the concentration of sodium chloride is 0.8 g/L and the concentration of potassium chloride is 0.2 g/L.

The assay and kit according to the present invention is useful in the determination of various analytes in various samples, in the art of clinical analysis, pharmaceutical analysis, food safety assessment, environmental monitoring, and the like.

Examples of the analyte include, but not limit to, microbes (such as viruses, bacteria, fungi), proteins, antibodies, antigens, biological toxins, chemical toxins, and the like.

Examples of the sample include, but not limit to, clinical samples, drug samples, water samples, biological samples, air samples, food samples, and beverage samples, etc.

The luminol directly bonded gold nanoparticle immunoprobe and the chemiluminescence immunoassay according to the present invention are illustrated in the following Examples 1-4, in which human IgG is detected using gold electrodes modified with gold nanoparticles linked to streptavidin as solid support, antibodies linked to biotin as capture probes, and electrochemiluminescence as the detection method.

Example 1. Preparation of the chemiluminescence immunoprobe
(1) 100 mL of 0.01 % (w/w) HAuCl₄ solution was heated to boil, and 1.5 mL of a solution containing 0.01 mol/L luminol and 0.01 mol/L NaOH was added under reflux with stirring. The reaction was kept boiling and stirred under reflux for 30 min. Then the heat source was removed and the reaction stirred for at least additional 15 min, resulting in a gold nanoparticle solution with a particle diameter of 25 nm. The solution was cooled to room temperature, and kept in dark below 4 °C.
(2) The synthesized gold nanoparticles solution was dialyzed against ultrapure water at room temperature for 2 days and the ultrapure water was exchanged once every 8 hours in order to remove free luminol and oxidation products so that luminol molecules in the gold nanoparticles were merely present in the form bonded to the surface of gold nanoparticles . Luminol directly bonded gold nanoparticles were thus obtained.
(3) 0.5 mL of 1.0 mg/mL secondary antibody (goat-anti-human IgG) was added to the dialyzed gold colloid solution in Step (2), for which pH was adjusted to 8.0 using 0.1 mol/L NaOH. The solution was incubated at room temperature for 30 min, and then 5 % (w/w) bovine serum albumin was added to the solution with constant stirring for 5 min, to the final concentration of 1%. Finally the solution was centrifuged at 17120 * g for 45 min so as to remove unreacted reagents and weakly bond luminol at the surface of gold nanoparticles. The precipitate was then dissolved in 0.1 mol/L pH 7.4 phosphate buffer containing 1 % (w/w) bovine serum albumin, obtaining luminol directly bonded gold nanoparticle immunoprobes (antibodies labeled with gold nanoparticles directly bond with luminol).

### Example 2. Immobilization of sandwich complexes onto a gold nanoparticle modified electrode

As shown in Figure 2, the immune sandwich complexes involved in chemiluminescent methods for immunoassay were immobilized on a gold nanoparticles modified electrode, in which the immobilization comprised:
(1) Preparation of the gold nanoparticles modified electrodes:
   A. A polished bare gold electrode was immersed into 2 mmol/L 1,3'-propanedithiol, and soaked at room temperature for 20 hours. They were taken out and rinsed by ethanol and ultrapure water, resulting in a 1,3'-propanedithiol modified gold electrode;
   B. 60 µL gold nanoparticles linked to streptavidin were added dropwise on the 1,3'-propanedithiol modified gold electrode prepared in Step A, and kept in dark at 4 °C for 4 hours, then taken out and rinsed by ultrapure water, resulting in a streptavidin / gold nanoparticles / 1,3'-propanedithiol modified gold electrode;
(2) Immobilization of sandwich complexes on the gold nanoparticles modified electrodes:
   a. Biotinlated primary antibody (goat-anti-human IgG) was added dropwise on the streptavidin / gold nanoparticle / 1,3'-propanedithiol modified gold electrode obtained in Step (2), kept at 37 °C for 60 min, and rinsed by 0.02 mol/L pH=7.4 phosphate buffer , resulting in a primary antibody / biotin / streptavidin / gold nanoparticle / 1,3'-propanedithiol modified gold electrode. Subsequently, 60 µl of 1% (w/w) bovine serum albumin was added dropwise to the primary antibody / biotin / streptavidin / gold nanoparticle / 1,3'-propanedithiol modified gold electrode, kept at 37 °C for 40 min, then rinsed in 0.02 mol/L pH=7.4 phosphate buffer.
   b. 60 µl of 7.5, 10, 20, 30, 40, 50, 70, 90, 100 pg/ml antigen (human IgG) was added dropwise on the primary antibody / biotin / streptavidin / gold nanoparticle / 1,3'-propanedithiol modified gold electrode obtained in Step a, kept at 37 °C for 60 min, and rinsed with 0.02 mol/L pH=7.4 phosphate buffer, resulting in an electrode modified with different concentrations of human IgG and the primary antibody, i.e. an antigen / primary antibody / biotin / streptavidin / gold nanoparticles / 1,3'-propanedithiol modified gold electrode.
   c. 60 µl of secondary antibody labeled by luminol directly bonded gold nanoparticles obtained in Example 1 (i.e. the immunoprobe) was added dropwise to the antigen / primary antibody / biotin / streptavidin / gold nanoparticle / 1,3'-propanedithiol modified gold electrodes obtained in Step b-3, kept at 37 °C for 60 min and rinsed in 0.02 mol/L pH=7.4 phosphate buffer, resulting in a modified electrode on which the secondary antibody labeled with luminol directly bonded gold nanoparticles specifically binds to analyte antigen (human IgG), i.e. the secondary antibody labeled with luminol directly bonded gold nanoparticles / antigen / primary antibody / biotin / streptavidin / gold nanoparticle / 1,3'-propanedithiol modified gold electrodes as shown in Figure 2.

### Example 3. Electrochemiluminescence immunoassay

The gold nanoparticle modified electrode linked to the sandwich immune complexes obtained in Example 2, i.e. the secondary antibody labeled with luminol directly bonded gold nanoparticle / antigen / primary antibody / biotin / streptavidin / gold nanoparticle / 1,3'-propanedithiol modified gold electrode, was placed in an electrochemiluminescence cuvette, and 0.02 mol/L pH=9.95 carbonate substrate solution containing 1 mmol/L peroxide hydrogen was added. Double-step pulse potential was applied with the pulse potential of 0.8 V, the pulse period of 30 s, the pulse time of 0.1 s, and the initial potential of 0 V, so as to produce electrochemiluminescence. Figure 3 shows curves of electrochemiluminescence upon time under double-step pulse potential on bare gold electrodes, 1,3'-propanedithiol modified gold electrodes, streptavidin/gold nanoparticles/1,3'-propanedithiol modified gold electrodes, and the secondary antibody labeled with luminol directly bonded gold nanoparticles / secondary antibody / antigen / primary antibody / biotin / streptavidin / gold nanoparticles / 1,3'-propanedithiol modified gold electrodes, in which the concentration of antigen is 100 pg/ml. The experimental results indicate that only the modified electrodes linked with luminol directly bonded gold nanoparticle immunoprobes produced electrochemiluminescence signals, while the bare gold electrodes, 1,3'-propanedithiol modified gold electrodes, or streptavidin/gold nanoparticles/1,3'-propanedithiol modified gold electrodes did not produce electrochemiluminescence signals .

Figure 4 shows the calibration curve for human IgG determination using the electrochemiluminescence immunoassay of the present invention. The results indicate that the linear range for human IgG is 7.5 - 100 pg/mL, and the detection limit is 1.0 pg/mL. The relative standard deviation of nine replicate determinations of hIgG was 4.4%, 2.1%, and 2.6% at 30, 50, and 70 pg/mL (n = 9), respectively.

### Example 4. Determination of IgG in actual human blood samples using the electrochemiluminescence immunoassay

The electrochemiluminescence immunoassay of the present invention was used to determine IgG in actual human blood samples. The results are shown in Table 1. The recoveries range from 106 % to 118.6 %, indicating that the immunoassay is applicable for determination human IgG in human serum samples. Table 2 shows the comparison between the chemiluminescence immunoassay of the present invention and turbidimetry method used in clinical analysis for the determination of IgG in human serum samples. The relatively deviation is less than 11.2 %, indicating that the results determined by the chemiluminescence immunoassay of the present invention are reliable.

| Table 1. Recoverry of IgG in actual human serum samples determined by the electrochemiluminescence immunoassay of the present invention | | | |
|---|---|---|---|
| The concentration of IgG in samples (pg/mL) | The concentration of IgG added (pg/mL) | The total concentration of IgG (pg/mL) (average ± deviation; n=5) | Recovery (%) |
| 11.33 | 50 | 64.32 ± 1.49 | 106.0 |
| 10.52 | 50 | 69.20 ± 1.97 | 117.4 |
| 9.74 | 50 | 67.37 ± 1.83 | 115.3 |
| 11.32 | 50 | 70.62 ± 1.34 | 118.6 |
| 10.39 | 50 | 65.17 ± 1.65 | 109.6 |
| Recovery = (The total concentration of IgG - the concentration of IgG in samples) / the concentration of IgG added× 100 %. | | | |
| Initial potential, 0 V; pulse period, 30 s; pulse time, 0.1 s ; pulse potential, 0.8 V; The composition of electrolyte: H₂O₂, 1.0 mM; CBS, 0.02 mM (pH 9.95). | | | |

| Table 2. The comparison between the chemiluminescence immunoassay of the present invention and turbidimetry method used in clinical analysis for the determination of IgG in human serum samples | | | |
|---|---|---|---|
| Samples | Clinical method (mg/mL) | The method of the present invention (mg/mL) (average ± deviation; n=5) | Relative deviation (%) |
| 1 | 10.62 | 11.33 ± 0.31 | 6.7 |
| 2 | 9.98 | 10.52 ± 0.69 | 5.4 |
| 3 | 10.97 | 9.74 ± 0.56 | 11.2 |
| 4 | 10.46 | 11.32 ± 0.53 | 8.2 |
| 5 | 9.63 | 10.39 ± 0.82 | 7.9 |
| Initial potential, 0V; pulse period, 30s; pulse time, 0.1 s; pulse potential, 0.8 V; The composition of the substrate solution: H₂O₂, 1.0 mM ; CBS, 0.02 mM (pH 9.95); the clinical method is turbidimetry method. | | | |

### Example 5. Chemiluminescence immunoassay using magnetic beads as solid support

The chemiluminescence immunoassay may also employ magnetic beads as the solid support. Conventional magnetic beads are mostly magnetic beads of iron oxide coated with polystyrene. The capture probes can be bound to magnetic beads by any known method for linkage of proteins and magnetic beads. These existing methods include, but not limit to, those reported in: Zhan, W.; Bard, A. J. Anal. Chem. 2007, 79, 459 ; Miao, W. J.; Bard, A. J. Anal. Chem. 2004, 76,7109; Yang, S. Y.; Lien, K. Y.; Huang, K. J. Biosens. Bioelectron. 2008, 24, 855; Selvaraju, T.; Das, J.; Han, S. W. Biosens. Bioelectron. 2008, 23, 932; Zhang, R. Q.; Hirakama, K.; Seto, D. Talanta. 2005, 68, 231. For example, proteins can bind directly to the magnetic beads, or through streptavidin and biotin. Thus, the capture probes can bind directly to the magnetic beads or through streptavidin and biotin. The magnetic beads with capture probes may further conjugate with the analyte of interest and analytical probes, to form sandwich immune complexes for chemiluminescence detection.

Magnetic beads modified with gold nanoparticles may also be used as the solid support. Firstly, the surface of magnetic beads can be modified with amino group (Weizmann, Y.; Patolsky, F.; Katz, E.; Willner, I. J. Am. Chem. Soc. 2003, 125, 3452), and gold nanoparticles are conjugated to the surface of magnetic beads through weak covalent bond between the amino group and the gold nanoparticles. The gold nanoparticles may be linked to the capture probes by any known method for the linkage of proteins and gold nanoparticles (Yin, X. B.; Qi, B.; Sun; X. P.; Yang; X. R.; Wang; E. K. Anal. Chem. 2004, 77, 3525 ; Gonzalez-Garcia, M. B.; Fernandez-Sanchez, C.; Costa-Garcia, A. Biosens. Bioelectron. 2000, 15, 315), for example, streptavidin and biotin. Subsequently, they are conjugated with the analyte of interest and analytical probes, forming sandwich immune complexes for chemiluminescence detection.

### Example 6. Chemiluminescence immunoassay using microplate as solid support

The chemiluminescence immunoassay may also employ microplate as the solid support. Conventional microplates are mostly made of polystyrene materials. The capture probes can be bound to microplates by any known method for the linkage of proteins and microplate. These existing methods include, but not limit to, those reported in: Duan, C. F.; Yu, Y. Q.; Cui, H. Analyst. 2008, 133, 1250; Piermarini, S.; Micheli, L.; Ammida, N. H. S. Biosens. Bioelectron. 2007, 22, 1434; Kiening, M.; Niessner, E. R.; Weller, M. G. Analyst. 2005, 130, 1580; R, M.; Cervino, C.; Sauceda, J. C.; Niessner, R.; Knopp, D. Anal. Chem. 2009, 81, 2373. For example, proteins can bind to the microplate directly, or through streptavidin and biotin. Thus, the capture probes can bind to microplate directly or through streptavidin and biotin. The microplate with capture probes may further conjugate with the analyte of interest and analytical probes, forming sandwich immune complexes for chemiluminescence detection.

Microplates modified with gold nanoparticles may be also used as the solid support. A layer of proteins such as bovine serum albumin (BSA) was firstly coated on the surface of the microplate, and gold nanoparticles were conjugated to microplate utilizing the property that gold nanoparticles could conjugate with proteins. Gold nanoparticles could be linked to capture probes by any known method for the linkage of proteins and gold nanoparticles (Yin, X. B.; Qi, B.; Sun; X. P.; Yang; X. R.; Wang; E. K. Anal. Chem. 2004, 77, 3525; Gonzalez-Garcia, M. B.; Fernandez-Sanchez, C.; Costa-Garcia, A. Biosens. Bioelectron. 2000, 15, 315), for example, streptavidin and biotin. Subsequently, they are conjugated with the analyte of interest and analytical probes, forming sandwich immune complexes for chemiluminescence detection.

### Example 7. Chemiluminescence immunoassay using nylon as solid support

The chemiluminescence immunoassay may also employ nylon as the solid support. Conventional nylon is mostly made of polyamides. The capture probes can be linked to nylon by any known method for the linkage of proteins and nylon. These existing methods comprise, but not limit to, those reported in: Rubtsova, M. Y.; Kovba, G. V.; Egorov, A. M. Biosens. Bioelectron. 1998, 13, 715; Zhang, F. H.; Yang, S. H.; Kang, T. Y.; Cha, G. S.; Nam, H.; Meyehoff, M. E. Biosens. Bioelectron. 2007, 22, 1419; Shah, J.; Chemburu, S.; Wilkins, E.; Abdel-Hamin, I. Electroanalysis. 2003, 15, 1809. For example, proteins can bind to nylon directly or through streptavidin and biotin. Thus, the capture probes can bind to nylon directly or through streptavidin and biotin. The nylon with capture probes may further conjugate with the analyte of interest and immunoprobes, forming sandwich immune complexes for chemiluminescence detection.

Nylon modified with gold nanoparticles may be also used as the solid support. Firstly, nylon was functionalized with amino group (Nan, C. F.; Zhang, Y.; Zhang, G. M.; Dong, C.; Shuang, S. M.; Choi, M. M-F. Enz. Microb. Technol. 2009, 44, 249), and gold nanoparticles are conjugated to the surface of nylon through the weak covalent bond between amino group and gold nanoparticles. The gold nanoparticles may be linked to capture probes by any known method for the linkage of proteins and gold nanoparticles (Yin, X. B.; Qi, B.; Sun; X. P.; Yang; X. R.; Wang; E. K. Anal. Chem. 2004, 77, 3525 ; Gonzalez-Garcia, M. B.; Fernandez-Sanchez, C.; Costa-Garcia, A. Biosens. Bioelectron. 2000, 15, 315), for example, streptavidin and biotin. Subsequently, they are conjugated with the analyte of interest and immunoprobes, forming sandwich immune complexes for chemiluminescence detection.

### Example 8. Chemiluminescence immunoassay using glass as solid support

The chemiluminescence immunoassay may also employ glass as the solid support. Conventional glass includes glass functionalized on the surface with aldehyde, amino or thiol group. The capture probes can be linked to glass by any known method for the linkage of proteins and glass. These existing methods comprise, but not limit to, those reported in: Yang, Z. J.; Xie, Z. Y; Liu, H.; Yan, F.; Ju, H. X. Adv. Funct. Mater. For example, proteins can bind to glass modified with polymers through streptavidin and biotin. Thus, the capture probes can bind to glass directly or through streptavidin and biotin. The glass with capture probes may further conjugate with the analyte of interest and immunoprobes, forming sandwich immune complexes for chemiluminescence detection.

Glass modified with gold nanoparticles may be also used as the solid support. Firstly, gold-plated glass may be modified with a layer of double thiol compounds (Wang, Z. P.; Hu, J. Q.; Jin, Y.; Yao, X.; Li, J. H. Clin. Chem. 2006, 52, 1958 ; ), and gold nanoparticles are conjugated to the surface of glass through the covalent bond between thiol group and gold nanoparticles. The gold nanopartiles may be linked to capture probes by any known method for the linkage of proteins and gold nanoparticles (Yin, X. B.; Qi, B.; Sun; X. P.; Yang; X. R.; Wang; E. K. Anal. Chem. 2004, 77, 3525; Gonzalez-Garcia, M. B.; Fernandez-Sanchez, C.; Costa-Garcia, A. Biosens. Bioelectron. 2000, 15, 315), for example streptavidin and biotin. Subsequently, they are conjugated with the analyte of interest and analytical probes, forming sandwich immune complexes for chemiluminescence detection.

The present invention is not limited to the embodiments described above, and the principle can be applied to the determination of various analytes. Any chemiluminescence immunoassay using luminol directly bonded gold nanoparticles as the luminescence label falls into the scope of the present invention. For example, changes in the linkage of the analyte of interest, in particle size and shape of the gold nanoparticles, in the materials of electrodes, in analytes, in antibodies, and the utilization of magnetic beads or microplates as solid support all belong to equivalent variations or modifications to the present invention.

Embodiments of the present invention may be understood by reference to the following numbered paragraphs:
1. A luminol directly bonded gold nanoparticle immunoprobe.
2. The luminol directly bonded gold nanoparticle immunoprobe according to paragraph 1, **characterized in that** the immunoprobe is an antibody or antigen or protein which is labeled with luminol directly bonded gold nanoparticles.
3. The luminol directly bonded gold nanoparticle immunoprobe according to paragraphs 1 or 2, **characterized in that** the luminol directly bonded gold nanoparticles are obtained in a one step synthesis process by the reduction of chlorauric acid with luminol.
4. The luminol directly bonded gold nanoparticle immunoprobe according to any one of paragraphs 1-3, **characterized in that** the immunoprobe is a chemiluminescence immunoprobe.
5. The luminol directly bonded gold nanoparticle immunoprobe according to paragraph 4, characterized in that the chemiluminescence immunoprobe is an eletrochemiluminescence immunoprobe.
6. A chemiluminescence immunoassay comprising the following steps:
   A, synthesizing the luminol directly bonded gold nanoparticle immunoprobe according to any one of paragraphs 1-5;
   B, incubating a capture probe with a solid support, such that the capture probe is linked onto the solid support, forming a capture probe/solid support complex;
   C, incubating the capture probe/solid support complex obtained in Step B with an analyte of interest, allowing the binding between the capture probe/solid support complex and the analyte of interest, forming an analyte of interest/capture probe/solid support complex;
   D, incubating the analyte of interest/capture probe/solid support complex obtained in Step C with the luminol directly bonded gold nanoparticle immunoprobe obtained in Step A, so as to form an immunoassay probe/analyte of interest/capture probe/solid support sandwich complex;
   E, adding the immunoassay probe/analyte of interest/capture probe/solid support sandwich complex obtained in Step D into a chemiluminescence cuvette, adding a substrate solution to produce chemiluminescence, detecting the chemiluminescence using a chemiluminescence analyzer.
7. The chemiluminescence immunoassay according to paragraph 6, **characterized in that** the chemiluminescence immunoassay is an electrochemiluminescence immunoassay.
8. The chemiluminescence immunoassay according to paragraphs 6 or 7, **characterized in that** the capture probe is an antibody or an antibody linked to a bridging molecule.
9. The chemiluminescence immunoassay according to paragraph 8, **characterized in that** the bridging molecule is biotin.
10. The chemiluminescence immunoassay according to paragraphs 6 or 7, **characterized in that** the solid support is selected from the group consisting of electrodes, magnetic beads, microplates, nylon, and glass.
11. The chemiluminescence immunoassay according to paragraph 10, **characterized in that** the electrode is an electrode modified with gold nanoparticles.
12. The chemiluminescence immunoassay according to paragraph 11, **characterized in that** the electrode modified with gold nanoparticles is selected from the group consisting of gold electrodes modified with gold nanoparticles, platinum electrodes modified with gold nanoparticles, vitreous carbon electrodes modified with gold nanoparticles, graphite electrodes modified with gold nanoparticles, wax-impregnated graphite electrodes modified with gold nanoparticles, and indium-tin oxide electrodes modified with gold nanoparticles.
13. The chemiluminescence immunoassay according to paragraph 12, **characterized in that** the bridging molecule used in the gold electrode modified with gold nanoparticles is selected from the group consisting of double-thiol compounds and compounds carrying an amino group and an thiol group, preferably the double-thiol compound is 1,3'-propanedithiol.
14. The chemiluminescence immunoassay according to any one of paragraphs 11-13, characterized in that the gold nanoparticle is linked to streptavidin.
15. The chemiluminescence immunoassay according to any one of paragraphs 11-13, characterized in that the particle size of the gold nanoparticle is within the range of 10-38 nm, preferably 16 nm.
16. The chemiluminescence immunoassay according to paragraphs 6 or 7, in which the chemiluminescence cuvette in Step E is an electrochemiluminescence cuvette, in which the sandwich immune complexes may produce chemiluminescence upon of addition of a substrate solution and application of potential.
17. The chemiluminescence immunoassay according to paragraph 16, **characterized in that** the substrate solution in Step E is carbonate buffer containing peroxide hydrogen.
18. The chemiluminescence immunoassay according to paragraph 16, **characterized in that** the applied potential used in Step E is pulse potential, cyclic voltammetry, or linear voltammetry.
19. The chemiluminescence immunoassay according to paragraph 18, in which the pulse potential is double-step pulse potential.
20. The chemiluminescence immunoassay according to paragraphs 6 or 7, **characterized in that** the analyte of interest is an antibody, an antigen, or a protein, and the sample containing the analyte of interest is derived from the group consisting of clinical samples, drug samples, water samples, biological samples, air samples, food samples and beverage samples.
21. A kit for the chemiluminescence immunoassay according to paragraphs 6-20, comprising:
   a. an immunoprobe according to any one of paragraphs 1-5;
   b. a capture probe;
   c. a solid support;
   d. a substrate solution;
   e. a washing solution.
22. The kit according to paragraph 21, characterized in that the immunoprobe is an antibody labeled with luminol directly bonded gold nanoparticles.
23. The kit according to paragraph 22, **characterized in that** the particle size of the gold nanoparticles is within the range of 14-25 nm, preferably 25 nm.
24. The kit according to paragraph 21, characterized in that the capture probe is an antibody labeled with biotin.
25. The kit according to paragraph 21, characterized in that the solid support is a gold electrode modified with gold nanoparticles.
26. The kit according to paragraph 25, characterized in that the gold electrode modified with gold nanoparticles is prepared by assembling gold nanoparticles on a gold electrode through 1,3'-propanedithiol.
27. The kit according to paragraph 26, characterized in that the gold nanoparticle is a gold nanoparticle linked to streptavidin.
28. The kit according to paragraph 27, **characterized in that** the particle size of the gold nanoparticle is within the range of 10-38 nm, preferably 16 nm.
29. The kit according to paragraph 21, characterized in that the substrate solution is carbonate buffer containing peroxide hydrogen, in which the carbonate salt consists of sodium carbonate and sodium bicarbonate.
30. The kit according to paragraph 29, characterized in that the concentration of peroxide hydrogen is 1 mmol/L.
31. The kit according to paragraph 29, characterized in that the concentrations of sodium carbonate and sodium bicarbonate are each 0.02 mol/L.
32. The kit according to paragraph 21, characterized in that the washing solution is phosphate buffer comprising potassium dihydrogen phosphate, disodium hydrogen phosphate, sodium chloride and potassium chloride.
33. The kit according to paragraph 32, characterized in that the concentration of phosphate buffer is 0.02 mol/L, in which the concentration of potassium dihydrogen phosphate is 0.2 g/L, the concentration of disodium hydrogen phosphate is 2.9 g/L, the concentration of sodium chloride is 0.8 g/L and the concentration of potassium chloride is 0.2 g/L.
34. The use of luminol directly bonded gold nanoparticles in preparation of immunoprobes.

## Claims

1. A luminol directly bonded gold nanoparticle immunoprobe, wherein the immunoprobe is an antibody or antigen or protein which is labeled with luminol directly bonded gold nanoparticles.

2. The luminol directly bonded gold nanoparticle immunoprobe according to claim 1, wherein the luminol directly bonded gold nanoparticles are obtained in a one step synthesis process by the reduction of chlorauric acid with luminol.

3. The luminol directly bonded gold nanoparticle immunoprobe according to claim 1 or claim 2, wherein the immunoprobe is a chemiluminescence immunoprobe, preferably an eletrochemiluminescence immunoprobe.

4. A chemiluminescence immunoassay comprising the following steps:
A, synthesizing the luminol directly bonded gold nanoparticle immunoprobe as defined in any one of claims 1 to 3;
B, incubating a capture probe with a solid support, such that the capture probe is linked onto the solid support, forming a capture probe/solid support complex;
C, incubating the capture probe/solid support complex obtained in Step B with an analyte of interest, allowing the binding between the capture probe/solid support complex and the analyte of interest, forming an analyte of interest/capture probe/solid support complex;
D, incubating the analyte of interest/capture probe/solid support complex obtained in Step C with the luminol directly bonded gold nanoparticle immunoprobe obtained in Step A, so as to form an immunoassay probe/analyte of interest/capture probe/solid support sandwich complex;
E, adding the immunoassay probe/analyte of interest/capture probe/solid support sandwich complex obtained in Step D into a chemiluminescence cuvette, adding a substrate solution to produce chemiluminescence, detecting the chemiluminescence using a chemiluminescence analyzer.

5. The chemiluminescence immunoassay according to claim 4, wherein the chemiluminescence immunoassay is an electrochemiluminescence immunoassay.

6. The chemiluminescence immunoassay according to claim 4 or claim 5, wherein the capture probe is an antibody or an antibody linked to a bridging molecule, the bridging molecule is preferably biotin.

7. The chemiluminescence immunoassay according to claim 4 or claim 5, wherein the solid support is selected from the group consisting of electrodes, magnetic beads, microplates, nylon, and glass, wherein the electrode is preferably an electrode modified with gold nanoparticles, preferably selected from the group consisting of gold electrodes modified with gold nanoparticles, platinum electrodes modified with gold nanoparticles, vitreous carbon electrodes modified with gold nanoparticles, graphite electrodes modified with gold nanoparticles, wax-impregnated graphite electrodes modified with gold nanoparticles, and indium-tin oxide electrodes modified with gold nanoparticles, and
wherein preferably the bridging molecule used in the gold electrode modified with gold nanoparticles is selected from the group consisting of double-thiol compounds and compounds carrying an amino group and an thiol group, preferably the double-thiol compound is 1,3'-propanedithiol.

8. The chemiluminescence immunoassay according to claim 7, wherein the gold nanoparticle used to modify the electrode is linked to streptavidin.

9. The chemiluminescence immunoassay according to claim 7, wherein the particle size of the gold nanoparticle used to modify the electrode is within the range of 10-38 nm, preferably 16 nm.

10. The chemiluminescence immunoassay according to claim 4 or claim 5, wherein the chemiluminescence cuvette in Step E is an electrochemiluminescence cuvette, in which the sandwich immune complexes may produce chemiluminescence upon of addition of a substrate solution and application of potential.

11. The chemiluminescence immunoassay according to claim 4 or claim 5, wherein the analyte of interest is an antibody, an antigen, or a protein, and the sample containing the analyte of interest is derived from the group consisting of clinical samples, drug samples, water samples, biological samples, air samples, food samples and beverage samples.

12. A kit for the chemiluminescence immunoassay according to any one of claims 4 to 11, comprising:
a. an immunoprobe according to any one of claims 1 to 3, preferably wherein the particle size of the gold nanoparticles is within the range of 14-25 nm, preferably 25 nm;
b. a capture probe, preferably an antibody labeled with biotin;
c. a solid support;
d. a substrate solution;
e. a washing solution.

13. The kit according to claim 12, wherein the solid support is a gold electrode modified with gold nanoparticles, preferably prepared by assembling gold nanoparticles on a gold electrode through 1,3'-propanedithiol, wherein the gold nanoparticle used to modify the electrode is preferably a gold nanoparticle linked to streptavidin, and the particle size of the gold nanoparticle is preferably within the range of 10-38 nm, preferably 16 nm.

14. The use of luminol directly bonded gold nanoparticles in the preparation of an immunoprobe, wherein the immunoprobe is an antibody or antigen or protein which is labeled with luminol directly bonded gold nanoparticles.

## Patentansprüche

1. Direkt an Luminol gebundene Goldnanopartikel-Immunsonde, wobei die Immunsonde ein Antikörper oder Antigen oder Protein ist, der bzw. das mit direkt an Luminol gebundenen Goldnanopartikeln markiert ist.

2. Direkt an Luminol gebundene Goldnanopartikel-Immunsonde nach Anspruch 1, wobei die direkt an Luminol gebundenen Goldnanopartikel in einem einstufigen Syntheseverfahren durch die Reduktion von Tetrachloridogoldsäure mit Luminol erhalten werden.

3. Direkt an Luminol gebundene Goldnanopartikel-Immunsonde nach Anspruch 1 oder Anspruch 2, wobei die Immunsonde eine Chemilumineszenz-Immunsonde, vorzugsweise eine Elektrochemilumineszenz-Immunsonde ist.

4. Chemilumineszenz-Immunassay, umfassend die folgenden Schritte:
A, Synthetisieren der direkt an Luminol gebundenen Goldnanopartikel-Immunsonde wie in einem der Ansprüche 1 bis 3 definiert;
B, Inkubieren einer Fängersonde mit einem festen Träger, so dass die Fängersonde auf dem festen Träger verknüpft ist, wobei ein Komplex aus Fängersonde/festem Träger gebildet wird;
C, Inkubieren des in Schritt B erhaltenen Komplexes aus Fängersonde/festem Träger mit einem interessierenden Analyten, wodurch die Bindung zwischen dem Komplex aus Fängersonde/festem Träger und dem interessierenden Analyten ermöglicht wird, wodurch ein Komplex aus interessierendem Analyten/Fängersonde/festem Träger gebildet wird;
D, Inkubieren des in Schritt C erhaltenen Komplexes aus interessierendem Analyten/Fängersonde/festem Träger mit der in Schritt A erhaltenen, direkt an Luminol gebundenen Goldnanopartikel-Immunsonde, um so einen Sandwich-Komplex aus Immunassaysonde/interessierendem Analyten/Fängersonde/festem Träger zu bilden;
E, Hinzufügen des in Schritt D erhaltenen Sandwich-Komplexes aus Immunassaysonde/interessierendem Analyten/Fängersonde/festem Träger zu einer Chemilumineszenzküvette, Hinzufügen einer Substratlösung, um Chemilumineszenz zu produzieren, Detektieren der Chemilumineszenz unter Verwendung eines Chemilumineszenzanalysators.

5. Chemilumineszenzimmunassay nach Anspruch 4, wobei der Chemilumineszenzimmunassay ein Elektrochemilumineszenzimmunassay ist.

6. Chemilumineszenzimmunassay nach Anspruch 4 oder Anspruch 5, wobei die Fängersonde ein Antikörper oder mit einem Brückenmolekül verknüpfter Antikörper ist, wobei das Brückenmolekül vorzugsweise Biotin ist.

7. Chemilumineszenzimmunassay nach Anspruch 4 oder Anspruch 5, wobei der feste Träger ausgewählt ist aus der Gruppe bestehend aus Elektroden, magnetischen Perlen, Mikroplatten, Nylon und Glas, wobei die Elektrode vorzugsweise eine mit Goldnanopartikeln modifizierte Elektrode ist, vorzugsweise ausgewählt aus der Gruppe bestehend aus mit Goldnanopartikeln modifizierten Goldelektroden, mit Goldnanopartikeln modifizierten Platinelektroden, mit Goldnanopartikeln modifizierten Glaskohlenstoffelektroden, mit Goldnanopartikeln modifizierten Graphitelektroden, mit Goldnanopartikeln modifizierten wachsimprägnierten Graphitelektroden und mit Goldnanopartikeln modifizierten Indium-Zinnoxid-Elektroden, und
wobei das Brückenmolekül, das in der mit Goldnanopartikeln modifizierten Goldelektrode verwendet wird, vorzugsweise ausgewählt ist aus der Gruppe bestehend aus Doppelthiolverbindungen und Verbindungen, die eine Aminogruppe und eine Thiolgruppe tragen, wobei die Doppelthiolverbindung vorzugsweise 1,3'-Propandithiol ist.

8. Chemilumineszenzimmunassay nach Anspruch 7, wobei das zum Modifizieren der Elektrode verwendete Goldnanopartikel mit Streptavidin verknüpft ist.

9. Chemilumineszenzimmunassay nach Anspruch 7, wobei die Partikelgröße des zum Modifizieren der Elektrode verwendeten Goldnanopartikels im Bereich von 10-38 nm, vorzugsweise 16 nm liegt.

10. Chemilumineszenzimmunassay nach Anspruch 4 oder Anspruch 5, wobei die Chemilumineszenzküvette in Schritt E eine Elektrochemilumineszenzküvette ist, in der die Sandwich-Immunkomplexe nach Hinzufügen einer Substratlösung und Anlegen eines Potentials Chemilumineszenz produzieren können.

11. Chemilumineszenzimmunassay nach Anspruch 4 oder Anspruch 5, wobei der interessierende Analyt ein Antikörper, ein Antigen oder ein Protein ist und die den interessierenden Analyten enthaltende Probe von der Gruppe bestehend aus klinischen Proben, Arzneimittel/Drogenproben, Wasserproben, biologischen Proben, Luftproben, Nahrungsmittelproben und Getränkeproben abgeleitet ist.

12. Kit für den Chemilumineszenzimmunassay nach einem der Ansprüche 4 bis 11, umfassend
a. eine Immunsonde nach einem der Ansprüche 1 bis 3, wobei vorzugsweise die Partikelgröße der Goldnanopartikel im Bereich von 14-25 nm, vorzugsweise 25 nm liegt;
b. eine Fängersonde, vorzugsweise einen mit Biotin markierten Antikörper;
c. einen festen Träger;
d. eine Substratlösung;
e. eine Waschlösung.

13. Kit nach Anspruch 12, wobei der feste Träger eine mit Goldnanopartikeln modifizierte Goldelektrode ist, vorzugsweise hergestellt durch Anfügen von Goldnanopartikeln an eine Goldelektrode mittels 1,3'-Propandithiol, wobei das zum Modifizieren der Elektrode verwendete Goldnanopartikel vorzugsweise ein mit Streptavidin verknüpftes Goldnanopartikel ist und die Partikelgröße des Goldnanopartikels vorzugsweise im Bereich von 10-38 nm, vorzugsweise 16 nm ist.

14. Verwendung von direkt an Luminol gebundenen Goldnanopartikeln zur Herstellung einer Immunsonde, wobei die Immunsonde ein Antikörper oder Antigen oder Protein ist, das mit direkt an Luminol gebundenen Goldnanopartikeln markiert ist.

## Revendications

1. Immunocapteur à base de nanoparticules d'or directement liées à du luminol, dans lequel l'immunocapteur est un anticorps, un antigène ou une protéine qui est marqué par des nanoparticules d'or directement liées à du luminol.

2. Immunocapteur à base de nanoparticules d'or directement liées à du luminol selon la revendication 1, dans lequel les nanoparticules d'or directement liées à du luminol sont obtenues dans un procédé de synthèse à une étape par la réduction de trichlorure d'or avec du luminol.

3. Immunocapteur à base de nanoparticules d'or directement liées à du luminol selon la revendication 1 ou la revendication 2, dans lequel l'immunocapteur est un immunocapteur à chimiluminescence, de préférence un immunocapteur à électrochimiluminescence.

4. Immuno-essai à chimiluminescence comprenant les étapes suivantes :
A, la synthèse de l'immunocapteur à base de nanoparticules d'or directement liées à du luminol tel que défini dans l'une quelconque des revendications 1 à 3 ;
B, l'incubation d'une sonde de capture avec un support solide, de sorte que la sonde de capture est liée sur un support solide, formant un complexe sonde de capture/support solide ;
C, l'incubation du complexe sonde de capture/support solide obtenu à l'étape B avec un analyte d'intérêt, permettant la liaison entre le complexe sonde de capture/support solide et l'analyte d'intérêt, formant un complexe analyte d'intérêt/sonde de capture/support solide ;
D, l'incubation du complexe analyte d'intérêt/sonde de capture/support solide obtenu à l'étape C avec l'immunocapteur à base de nanoparticules d'or directement liées à du luminol obtenu à l'étape A, de manière à former un complexe sonde d'immuno-essai/analyte d'intérêt/sonde de capture/support solide en sandwich ;
E, l'ajout du complexe sonde d'immuno-essai/analyte d'intérêt/sonde de capture/support solide en sandwich obtenu à l'étape D dans une cuvette de chimiluminescence, l'ajout d'une solution de substrat pour produire une chimiluminescence, la détection de la chimiluminescence à l'aide d'un analyseur à chimiluminescence.

5. Immuno-essai à chimiluminescence selon la revendication 4, dans lequel l'immuno-essai à chimiluminescence est un immuno-essai à électrochimiluminescence.

6. Immuno-essai à chimiluminescence selon la revendication 4 ou la revendication 5, dans lequel la sonde de capture est un anticorps ou un anticorps lié à une molécule de pontage, la molécule de pontage étant de préférence la biotine.

7. Immuno-essai à chimiluminescence selon la revendication 4 ou la revendication 5, dans lequel le support solide est choisi parmi le groupe constitué d'électrodes, de billes magnétiques, de microplaques, de nylon et de verre, dans lequel l'électrode est de préférence une électrode modifiée avec des nanoparticules d'or, de préférence choisies parmi le groupe constitué d'électrodes d'or modifiées avec des nanoparticules d'or, des électrodes de platine modifiées avec des nanoparticules d'or, des électrodes de carbone vitreux modifiées avec des nanoparticules d'or, des électrodes de graphite modifiées avec des nanoparticules d'or, des électrodes de graphite imprégné de cire modifiées avec des nanoparticules d'or et des électrodes d'oxyde d'indium et de tin modifiées avec des nanoparticules d'or, et
dans lequel de préférence la molécule de pontage utilisée dans l'électrode d'or modifiée avec des nanoparticules d'or est choisie parmi le groupe constitué de composés à thiol double et de composés porteurs d'un groupe amino et d'un groupe thiol, le composé à thiol double étant de préférence le 1,3'-propanedithiol.

8. Immuno-essai à chimiluminescence selon la revendication 7, dans lequel la nanoparticule d'or utilisée pour modifier l'électrode est liée à la streptavidine.

9. Immuno-essai à chimiluminescence selon la revendication 7, dans lequel la taille de la nanoparticule d'or utilisée pour modifier l'électrode se situe dans la plage de 10 à 38 nm, de préférence 16 nm.

10. Immuno-essai à chimiluminescence selon la revendication 4 ou la revendication 5, dans lequel la cuvette de chimiluminescence de l'étape E est une cuvette d'électrochimiluminescence, dans laquelle les complexes immunes en sandwich peuvent produire une chimiluminescence par ajout d'une solution de substrat et application d'un potentiel.

11. Immuno-essai à chimiluminescence selon la revendication 4 ou la revendication 5, dans lequel l'analyte d'intérêt est un anticorps, un antigène ou une protéine, et l'échantillon contenant l'analyte d'intérêt est dérivé du groupe constitué d'échantillons cliniques, d'échantillons de médicaments, d'échantillons d'eau, d'échantillons biologiques, d'échantillons d'air, d'échantillons alimentaires et d'échantillons de boissons.

12. Trousse pour l'immuno-essai à chimiluminescence selon l'une quelconque des revendications 4 à 11, comprenant :
a. un immunocapteur selon l'une quelconque des revendications 1 à 3, de préférence dans laquelle la taille des nanoparticules d'or se situe dans la plage de 14 à 25 nm, de préférence 25 nm ;
b. une sonde de capture, de préférence un anticorps marqué à la biotine ;
c. un support solide ;
d. une solution de substrat ;
e. une solution de lavage.

13. Trousse selon la revendication 12, dans laquelle le support solide est une électrode d'or modifiée avec des nanoparticules d'or, de préférence préparée par assemblage de nanoparticules d'or sur une électrode d'or par du 1,3'-propanedithiol, dans laquelle la nanoparticule d'or utilisée pour modifier l'électrode est de préférence une nanoparticule d'or liée à la streptavidine, et la taille de la nanoparticule d'or se situe de préférence dans la plage de 10 à 38 nm, de préférence 16 nm.

14. Utilisation d'un immunocapteur à base de nanoparticules d'or directement liées à du luminol dans la préparation d'un immunocapteur, dans lequel l'immunocapteur est un anticorps, un antigène ou une protéine qui est marqué par des nanoparticules d'or directement liées à du luminol.
